# EUROPEAN PATENT SPECIFICATION

(11) **EP 1 440 152 B1**
(45) Date of publication and mention of the grant of the patent: **24.04.2013**
(21) Application number: 02772604.1
(22) Date of filing: 04.11.2002
(51) Int. Cl.: C12N 15/13, C12N 15/62, G01N 33/50, A61K 39/395, C07K 16/28

(54) **5T4 LIGAND**
5T4 LIGAND
5T4 LIGAND

(30) Priority: 02.11.2001 GB 0126378
(43) Date of publication of application: 28.07.2004
(73) Proprietor: Oxford Biomedica (UK) Limited, Oxford OX4 4GA (GB)
(72) Inventor: CARROLL, Miles, Oxford BioMedica (UK) Limited, The Oxford Science Park, Oxford OX4 4GA (GB); LAMIKANRA, Abigail, Oxford BioMedica (UK) Limited, The Oxford Science Park, Oxford OX4 4GA (GB); KINGSMAN, Alan, Oxford BioMedica (UK) Limited, The Oxford Science Park, Oxford OX4 4GA (GB)
(74) Representative: Harding, Charles Thomas
(86) International application number: PCT/GB2002/004965
(87) International publication number: WO 2003/038098

(56) References cited:
- WO-A-01/36486
- MYERS K A ET AL: "TARGETING IMMUNE EFFECTOR MOLECULES TO HUMAN TUMOR CELLS THROUGH GENETIC DELIVERY OF 5T4-SPECIFIC SCFV FUSION PROTEINS" CANCER GENE THERAPY, NORWALK, CT, US, vol. 9, no. 11, November 2002 (2002-11), pages 884-896, XP009007160 ISSN: 0929-1903
- FORSBERG G ET AL: "THERAPY OF HUMAN NON-SMALL-CELL LUNG CARCINOMA USING ANTIBODY TARGETING OF A MODIFIED SUPERANTIGEN" BRITISH JOURNAL OF CANCER, LONDON, GB, vol. 85, no. 1, 6 July 2001 (2001-07-06), pages 129-136, XP001145660 ISSN: 0007-0920
- SHAW^A D M ET AL: "Isolation of a high affinity scFv from a monoclonal antibody recognising the oncofoetal antigen 5T4" BBA - GENERAL SUBJECTS, ELSEVIER SCIENCE PUBLISHERS, NL, vol. 1524, no. 2-3, 15 December 2000 (2000-12-15), pages 238-246, XP004275928 ISSN: 0304-4165 cited in the application
- MYERS K A ET AL: "ISOLATION OF A CDNA ENCODING 5T4 ONCOFETAL TROPHOBLAST GLYCOPROTEIN" JOURNAL OF BIOLOGICAL CHEMISTRY, AMERICAN SOCIETY OF BIOLOGICAL CHEMISTS, BALTIMORE, MD, US, vol. 269, no. 12, 25 March 1994 (1994-03-25), pages 9319-9324, XP002064468 ISSN: 0021-9258
- VELDEN VAN DER V ET AL: "Targeting of the CD33-calicheamicin immunoconjugate Mylotarg (CMA-676) in acute myeloid leukemia: in vivo and in vitro saturation and internalization by leukemic and normal myeloid cells", Blood, vol. 97, no. 10, 15 May 2001 (2001-05-15), pages 3197-3204, XP009080680, Washington, USA
- DATABASE BIOSIS [Online] BIOSCIENCES INFORMATION SERVICE, PHILADELPHIA, PA, US 16 November 2000 BERNT K ET AL: 'CD19 targeted calicheamicin THETA is effective against high risk ALL' Database accession no. PREV200100330624
- BOGHAERT E R ET AL: "The oncofetal protein, 5T4, is a suitable target for antibody-guided anti-cancer chemotherapy with calicheamicin", INTERNATIONAL JOURNAL OF ONCOLOGY, vol. 32, no. 1, January 2008 (2008-01), pages 221-234, ISSN: 1019-6439
- SANDVIG K ET AL: "Entry of ricin and Shiga toxin into cells: molecular mechanisms and medical perspectives", EMBO JOURNAL, OXFORD UNIVERSITY PRESS, SURREY, GB, vol. 19, no. 22, 15 November 2000 (2000-11-15), pages 5943-5950, XP002228667, ISSN: 0261-4189, DOI: 10.1093/EMBOJ/19.22.5943
- SVETLANA O. DORONINA ET AL: 'Enhanced Activity of Monomethylauristatin F through Monoclonal Antibody Delivery: Effects of Linker Technology on Efficacy and Toxicity' BIOCONJUGATE CHEMISTRY vol. 17, no. 1, 01 January 2006, pages 114 - 124, XP055009264 ISSN: 1043-1802
- TSUTA K ET AL: "Using the Mitosis-Specific Marker Anti-Phosphohistone H3 to Assess Mitosis in Pulmonary Neuroendocrine Carcinomas", AMERICAN JOURNAL OF CLINICAL PATHOLOGY, vol. 136, August 2011 (2011-08), pages 252-259, ISSN: 0002-9173, DOI: 10.1309/AJCPDXFOPXGEF0RP

## Description

The present invention relates to a novel method for the delivery of agents to tumour cells. In particular it relates to a method for the specific delivery of agents to the interior of tumour cells. Uses of the method are also described.

### Background to the invention

A number of oncofoetal or tumour-associated antigens (TAAs) have been identified and characterised in human and animal tumours. In general, TAAs are antigens expressed during foetal development which are down regulated in adult cells, and are thus normally absent or present only at very low levels in adults. Tumour cells have been observed to resume expression of TAAs, and the application of TAAs for tumour diagnosis, targeting and immunotherapy has therefore been suggested.

Some TAAs are found to be regularly associated with tumours in a large number of individuals.

The TAA 5T4 (see WO 89/07947) has been extensively characterised. It is a 72kDa glycoprotein expressed widely in carcinomas, but having a highly restricted expression pattern in normal adult tissues (see Table 1). It appears to be strongly correlated to metastasis in colorectal and gastric cancer. The full nucleic acid sequence of human 5T4 is known (Myers et al., 1994 J Biol Chem 169: 9319-24).

**Table 1**

| **Distribution of Human 5T4 Tumour Type 5T4 Frequency (%)** | |
|---|---|
| Breast | 84 |
| Ovarian | 71 |
| Gastric | 74 |
| Colorectal | 85 |

(Starzynska et al., Eur J Gastroenterol Hepatol 1998 Jun;10(6):479-84; Starzynska et al., Br J Cancer 1994 May;69(5):899-902; Starzynska et al., Br J Cancer 1992 Nov;66(5):867-9)

5T4 antigen is expressed on the surface of cells. Likewise any ligand which binds to 5T4 antigen is also restricted to the surface of cells, and therefore although potentially useful as a marker, or in diagnosis, or imaging of tumour cells, until the present invention it was thought to have little use in the delivery of agents to the interior of cells.

### Summary of the invention

The present inventors have surprisingly found that the 5T4 antigen is capable of internalisation when a 5T4 antibody is bound. Thus, upon binding of a 5T4 antibody to the cell surface tumour antigen 5T4, the complex is internalised. This provides an effective means for the delivery of agents to the interior of cells, in particular the cell cytoplasm.

Thus, in a first aspect, the present invention provides a 5T4 antibody linked to a cytotoxic agent, which, once internalised into the interior of a tumour cell, exerts one or more intracellular biological effects, wherein the cytotoxic agent comprises a molecule which affects the cell structure by interacting with one or more cytoskeletal elements.

In a further aspect, the present invention provides a cell surface complex of 5T4 antigen and such a 5T4 antibody linked to an agent.

In a further aspect, the present invention provides an internalised complex of such a 5T4 antibody linked to an agent.

In the context of the present invention the term 'intracellular biological effects' means that the agent affects intracellular structure and/or function. One skilled in the art will appreciate that it is the agent which exerts the intracellular biological effects, either in isolation or when combined with the 5T4 antibody according to the present invention.

The term 'internalisation' means internalisation or movement into the interior of the cell, preferably the cytoplasm. Likewise an 'internalised complex' is one which has been moved from the exterior cell surface to the interior of the cell.

The term 'specific binding' in the context of the present invention means that such binding is not inhibited by the presence of non-related molecules. Likewise, the term 'a specific' 5T4 antibody describes an antibody which binds to 5T4 specifically.

The antibody may be a complete antibody, an antibody fragment, an antibody peptide or a bi-specific antibody. Thus, by way of example, an antibody may include Fv, ScFv, Fab' and F(ab')₂, monoclonal and polyclonal antibodies, engineered antibodies including chimeric, CDR-grafted and humanised antibodies, artificially selected antibodies produced using phage display or alternative techniques or peptides that bind to the cell surface tumour antigen 5T4. If the antibody is a bi-specific antibody then it may be able to bind to the 5T4 antigen and to an agent according to the present invention.

Preferably, the antibody is a monoclonal antibody. In an alternative embodiment of the above aspects of the invention, it is an scFv. Preferably the scFv is derived from a monoclonal antibody. Advantageously, the antibody is specific to human 5T4. In especially preferred embodiments of the invention, the antibody is 5T4scFvmycHis (scFv derived from monoclonal H8) and/or H8 monoclonal antibody.

The complex may form on the surface of a cell either *in vitro* or *in vivo.* In an especially preferred embodiment of this aspect of the invention, the complex forms on the surface of a cell *in vivo.*

In a further aspect, the present invention provides such a 5T4 antibody linked to an agent for use in a method for the internalisation of a 5T4 antibody linked to an agent into tumour cells by treating those one or more cells with a specific 5T4 ligand.

In a preferred embodiment of this aspect of the invention, internalisation occurs into the cytoplasm of cells.

In the context of the present invention, the term 'treating' refers to the process of bringing one or more cells into contact with a specific 5T4 antibody. Suitable methods for 'treating' cells will depend upon whether the procedure is carried out *in vivo* or *in vitro* and are described herein. In a preferred embodiment of this aspect of the invention, the method occurs *in vivo.*

Preferably, the tumour cells are any one or more selected from the group consisting of: breast tumour cells, ovarian tumour cells, gastric tumour cells and colorectal tumour cells.

In a further aspect, the present invention provides such a 5T4 antibody linked to an agent for use in a method for the specific delivery of one or more agents to the interior of tumour cells.

Advantageously, the specific delivery is to the cytoplasm. In a preferred aspect of the invention, the method is performed *in vivo.* That is, it is for the delivery of agents to the interior of tumour cells within a mammal. Advantageously, the mammal is a human. Suitable antibodies are any of those described herein. Advantageously, the 5T4 antibody is 5T4scFvmycHis and/or H8 as herein described.

In the context of the present invention, the term 'specific' delivery means that in a given population of cells an agent will only be delivered to the interior of a proportion of those cells. In this case, those cells display a 5T4 antigen (ie only tumour cells).

Suitable cytotoxic 'agents' for delivery to cells may be synthetic or naturally occurring. They may comprise proteins, peptides and/or nucleic acids. They comprise molecules which affects the cell structure by interacting with one or more cytoskeletal elements. They may have an effect on the structure or function of the cell. For instance they may have effects on cell proliferation.

In addition, the agent may comprise a nucleic acid sequence encoding a product, which may be of use therapeutically.

In addition, the agents may comprise components, which permit the subsequent detection, and/or imaging of tumour cells either *in vitro* or *in vivo.* Suitable components include radiolabels as herein described, suitable for detection by autoradiography. They also include MRI (magnetic resonance imaging labels). Labels suitable for detection by positron emission tomography are also included within the scope of the present invention. Those skilled in the art will be aware of other suitable agents.

Agents may be linked to a 5T4 antibody physically by for instance chemical crosslinking using agents such as iodoacetamide. Alternatively they may be expressed conjugated to a 5T4 antibody. For instance they may be expressed as fusion proteins. They may be attached to the 5T4 antibody via a 'linker sequence' for instance a peptide linker sequence. In this case the agent may not be in physical association with the 5T4 antibody. Alternatively, the agent may bind to the 5T4 antibody via the antigen binding site. Those skilled in the art will appreciate that this list is not intended to be exhaustive, and will be aware of other suitable methods of linking the agent to a 5T4 antibody of the present invention.

Advantageously, the tumour cells are selected from the group consisting of breast tumour cells, ovarian tumour cells, gastric tumour cells and colorectal tumour cells.

In a further aspect, the present invention provides such a 5T4 antibody linked to an agent for use in a method for decreasing the rate of proliferation of tumour cell/s.

Preferably, the 5T4 antibody is a monoclonal antibody or scFv. Advantageously, the 5T4 antibody is 5T4scFvmycHis and/or H8.

Preferably, the 5T4 is human 5T4.

A 'cytotoxic agent' in the context of the present invention refers to an agent which induces the onset or progression of cell senescence in at least a proportion of those cells when administered to a population of cells.

In the context of the present invention, the term 'decreasing the rate of proliferation of tumour cells' means that the rate of proliferation of a sample of tumour cells treated with a 5T4 antibody, as herein described, linked to a cytoxic agent is decreased when compared with a similar sample of cells which have not been so treated.

The method may be an *in vitro* or an *in vivo* method. Preferably, the method is an *in vivo* method. Advantageously, the method is decreasing the rate of proliferation of human tumour cells. Preferably the tumour cells are those selected from the following: breast tumour cells, ovarian tumour cells, gastric tumour cells and colorectal tumour cells.

In a further aspect the present invention provides such a 5T4 antibdoy linked to an agent for use in a method for the prophylaxis or treatment of cancer in a mammal.

Preferably, the cancer is of breast cancer, ovarian cancer, gastric cancer and colorectal cancer.

Common characteristics of 'cancer' as herein defined include the ability of a cell to undergo endless replication, loss of contact inhibition, invasiveness and the ability to metastasise. That is, when the cell divides in an uncontrollable way and can not recognise its own natural boundary, the cancer cells obtain the ability to spread to other areas of the body. Mutations within the nucleic acid of one or more cells are involved in the onset of cancer. Often, more than one nucleic acid mutation or other aberrant cellular event is required for the development of tumours (bundles of aberrantly dividing cells), that is tumour formation is a multi-signal event. In the context of the present invention cancer cells include any cells which exhibit any one or more of the following features aberrant cell division, aberrant contact inhibition, aberrant cell differentiation as compared with cells behaving normally within their native environment, the ability of the cell to invade tissues, and the ability to metastasise. The definition of 'cancer cells' in the context of the present invention, therefore includes within its scope tumour cells and also cells prior to the formation of tumours in so far as they possess one or more of the requisite characteristics listed above. In addition the term cancer cells according to the present invention includes metastatic cells.

### Brief description of the figures

Figure 1: Internalisation of hu5T4 on the cell surface of the murine cell line CT26hu5T4.
Figure 2: Internalisation of hu5T4 on the cell surface of the human cell line HT29 and PA1.
Figure 3: Internalisation of hu5T4 on the surface of MDAMB-4355T4. A decrease in 5T4 is detected at the surface of MDAMB-4355T4 following incubation with an antibody specific to 5T4.
Figure 4: Internalisation of hu5T4 on the surface of CHO-5T4EKmycHIS. A decrease in 5T4 is detected at the surface of CHO-hu5T4following incubation with an antibody specific to 5T4.

### Detailed description of the invention

Unless defined otherwise, all technical and scientific terms used herein have the same meaning as commonly understood by one of ordinary skill in the art (e.g., in cell culture, molecular genetics, nucleic acid chemistry, hybridisation techniques and biochemistry). The present invention employs, unless otherwise indicated, conventional techniques which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

### 5T4

Surprisingly, the inventors have now discovered that 5T4 is capable of internalisation when a 5T4 ligand is bound.

5T4 antigen is the polypeptide known as 5T4 and characterised, for example, in WO89/07947. In a preferred aspect, 5T4 is human 5T4 as characterised by Myers *et al ibid*., the sequence of which appears in GenBank at accession no. Z29083. The definition of 5T4 according to the present invention includes species and allelic variations of 5T4, including canine 5T4, as well as fragments, preferably distinct epitopes, and variants thereof comprising amino acid insertions, deletions or substitutions which retain the antigenicity of 5T4. Such fragments and variants are described in greater detail below.

In the context of the present invention the term '5T4 antigen' also includes within its scope a modified 5T4 antigen. A "modified" antigen, as used herein, is a 5T4 polypeptide, which has been truncated, extended or otherwise mutated such that it differs from naturally-occurring 5T4. It has been found that peptide fragments derived from 5T4 are able to function as 5T4-specific antigenic determinants. Such peptides are able to bind HLA molecules and to induce CTL responses against wild-type 5T4 in subjects, often more effectively that full-length 5T4. Moreover, 5T4 peptides may be mutated, by amino acid insertion, deletion or substitution; mutated peptides advantageously bind even more effectively to HLA and elicit an even more potent CTL response in subjects. Peptides may be any length, but are advantageously between 5 and 25 amino acids, preferably between 6 and 15 amino acids, and advantageously about 9 amino acids in length.

Modified peptides are advantageously HLA CTL epitopes of 5T4. Modification of such epitopes may be performed based on predictions for more efficient CTL induction derived using the program "Peptide Binding Predictions" devised by K. Parker (NIH) which may be found at http://www-bimas.dcrt.nih.gov/cgi-bin/molbio/ken parker comboform (see also Parker, K. C et al. (1994) J. Immunol. 152:163).

5T4 antigen, as referred to herein, includes peptides and other fragments of 5T4 which retain at least one common antigenic determinant of 5T4.

"Common antigenic determinant" means that the derivative in question has at least one antigenic function of 5T4. Antigenic function includes possession of an epitope or antigenic site that is capable of cross-reacting with antibodies raised against a naturally occurring or denatured 5T4 polypeptide or fragment thereof, or the ability to bind HLA molecules and induce a 5T4-specific immune response. Thus 5T4 as provided by the present invention includes splice variants encoded by mRNA generated by alternative splicing of a primary transcript, amino acid mutants, glycosylation variants and other covalent derivatives of 5T4 which retain the physiological and/or physical properties of 5T4. Exemplary derivatives include molecules wherein the protein of the invention is covalently modified by substitution, chemical, enzymatic, or other appropriate means with a moiety other than a naturally occurring amino acid. Such a moiety may be a detectable moiety such as an enzyme or a radioisotope. Further included are naturally occurring variants of 5T4 found with a particular species, preferably a mammal. Such a variant may be encoded by a related gene of the same gene family, by an allelic variant of a particular gene, or represent an alternative splicing variant of the 5T4 gene.

Derivatives which retain common antigenic determinants can be fragments of 5T4. Fragments of 5T4 comprise individual domains thereof, as well as smaller polypeptides derived from the domains. Preferably, smaller polypeptides derived from 5T4 according to the invention define a single epitope which is characteristic of 5T4. Fragments may in theory be almost any size, as long as they retain one characteristic of 5T4. Preferably, fragments will be between 5 and 400 amino acids in length. Longer fragments are regarded as truncations of the full-length 5T4 and generally encompassed by the term "5T4". Advantageously, fragments are relatively small peptides of the order of 5 to 25 amino acids in length. Preferred are peptides about 9 amino acids in length.

Derivatives of 5T4 also comprise mutants thereof, which may contain amino acid deletions, additions or substitutions, subject to the requirement to maintain at least one feature characteristic of 5T4. Thus, conservative amino acid substitutions may be made substantially without altering the nature of 5T4, as may truncations from the 5' or 3' ends. Deletions and substitutions may moreover be made to the fragments of 5T4 comprised by the invention. 5T4 mutants may be produced from a DNA encoding 5T4 which has been subjected to *in vitro* mutagenesis resulting e.g. in an addition, exchange and/or deletion of one or more amino acids. For example, substitutional, deletional or insertional variants of 5T4 can be prepared by recombinant methods and screened for immuno-crossreactivity with the native forms of 5T4.

Moreover, variant peptides can be screened for superior HLA binding capabilities using the program "Peptide Binding Predictions" devised by K. Parker at the National Institutes of Health (see Parker, K. C et al. 1994.J.Immunol. 152:163).

The fragments, mutants and other derivatives of 5T4 preferably retain substantial homology with 5T4. As used herein, "homology" means that the two entities share sufficient characteristics for the skilled person to determine that they are similar in origin and function. Preferably, homology is used to refer to sequence identity.

"Substantial homology", where homology indicates sequence identity, means more than 40% sequence identity, preferably more than 45% sequence identity and most preferably a sequence identity of 50% or more, as judged by direct sequence alignment and comparison.

Sequence homology (or identity) may moreover be determined using any suitable homology algorithm, using for example default parameters. Advantageously, the BLAST algorithm is employed, with parameters set to default values. The BLAST algorithm is described in detail at http://www.ncbi.nih.gov/BLAST/blast_help.html, which is incorporated herein by reference.

Advantageously, "substantial homology" when assessed by BLAST equates to sequences which match with an EXPECT value of at least about 7, preferably at least about 9 and most preferably 10 or more. The default threshold for EXPECT in BLAST searching is usually 10.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul (see http://www.ncbi.nih.gov/BLAST/blast_help.html) with a few enhancements. The BLAST programs were tailored for sequence similarity searching, for example to identify homologues to a query sequence. The programs are not generally useful for motif-style searching. For a discussion of basic issues in similarity searching of sequence databases, see Altschul et al. (1994) Nature Genetics 6:119-129. Most preferably, sequence comparisons are conducted using the simple BLAST search algorithm provided at http://www.ncbi.nlm.nih.gov/BLAST.

Alternatively, sequence homology may be determined by algorithms such as FastA, available at http://biology.ncsa.uiuc.edu/BW30/BW.cgi. FastA is considered to be superior to BLAST for alignment of short sequences. Advantageously, the FastA algorithm is employed using default parameters at http://biology.ncsa.uiuc.edu/BW30/BW.cgi.

### Antibodies which specifically bind to 5T4 antigen

According to the present invention, upon binding of a 5T4 antibody to 5T4, the complex is internalised.

As used herein, the term 'specific' binding means that such binding is not competitively inhibited by the presence of non-related molecules.

The 5T4 antibody may be a complete antibody or antibody fragment.

The antibody may be used in combination with one or more pharmaceutically active compounds, which may be administered simultaneously, separately or sequentially.

As used herein, the term "antibody" refers to complete antibodies, bi-specific antibodies or antibody fragments capable of binding to 5T4 as herein defined, and includes Fv, ScFv, Fab' and F(ab')₂, monoclonal and polyclonal antibodies, engineered antibodies including chimeric, CDR-grafted and humanised antibodies, and artificially selected antibodies produced using phage display or alternative techniques.

A chimeric antibody refers to a genetically engineered fusion of parts of a mouse antibody with parts of a human antibody. Generally, chimeric antibodies contain approximately 33 % mouse protein and 67 % human protein. Developed to reduce the HAMA response elicited by murine antibodies, they combine the specificity of the murine antibody with the efficient human immune system interaction of a human antibody.

Humanised antibodies have been obtained by replacing the constant region of a mouse antibody with human protein, but by also replacing portions of the antibody's variable region with human protein. Generally humanised antibodies are 5-10% mouse and 90-95% human. Humanised antibodies were developed to counter the immune responses seen with murine and chimeric antibodies. Data from humanised antibodies used in clinical trials show that humanised antibodies exhibit minimal or no response of the human immune system against them.

A more sophisticated approach to humanised antibodies involves not only providing human-derived constant regions, but also modifying the variable regions as well. This allows the antibodies to be reshaped as closely as possible to the human form. The variable regions of both heavy and light chains contain three complementarity-determining regions (CDRs) which vary in response to the antigens in question and determine binding capability, flanked by four framework regions (FRs) which are relatively conserved in a given species and which putatively provide a scaffolding for the CDRs. When non-human antibodies are prepared with respect to a particular antigen, the variable regions can be "reshaped" or "humanised" by grafting CDRs derived from non-human antibody on the FRs present in the human antibody to be modified. This approach has been reported in, for example, Cancer Res (1993) 53:851-856, Nature (1988) 332:323-327, Science (1988) 239:1534-1536, Proc Natl Acad Sci USA (1991) 88:4181-4185 and J Immunol (1992) 148:1149-1154.

Advantageously antibodies for use according to the present invention are H8 monoclonal, which is a monoclonal antibody recognising the oncofoetal antigen 5T4 and is described in Biochim Biophys Acta, (2000), Dec 15, 1524 (2-3):238-46 which is herein incorporated by reference and/or 5T4scFvmycHis scFv derived from the monoclonal which is described WO 00/29428 which is also herein incorporated by reference.

### Preparation of antibodies

Preparation of antibodies is performed using standard laboratory techniques Antibodies may be obtained from animal serum, or, in the case of monoclonal antibodies or fragments thereof, produced in cell culture. Recombinant DNA technology may be used to produce the antibodies according to established procedure, in bacterial or preferably mammalian cell culture. The selected cell culture system preferably secretes the antibody product.

Multiplication of hybridoma cells or mammalian host cells *in vitro* is carried out in suitable culture media, which are the customary standard culture media, for example Dulbecco's Modified Eagle Medium (DMEM) or RPMI 1640 medium, optionally replenished by a mammalian serum, e.g. foetal calf serum, or trace elements and growth sustaining supplements, e.g. feeder cells such as normal mouse peritoneal exudate cells, spleen cells, bone marrow macrophages, 2-aminoethanol, insulin, transferrin, low density lipoprotein, oleic acid, or the like. Multiplication of host cells which are bacterial cells or yeast cells is likewise carried out in suitable culture media known in the art, for example for bacteria in medium LB, NZCYM, NZYM, NZM, Terrific Broth, SOB, SOC, 2 x YT, or M9 Minimal Medium, and for yeast in medium YPD, YEPD, Minimal Medium, or Complete Minimal Dropout Medium.

*In vitro* production provides relatively pure antibody preparations and allows scale-up to give large amounts of the desired antibodies. Techniques for bacterial cell, yeast or mammalian cell cultivation are known in the art and include homogeneous suspension culture, e.g. in an airlift reactor or in a continuous stirrer reactor, or immobilised or entrapped cell culture, e.g. in hollow fibres, microcapsules, on agarose microbeads or ceramic cartridges.

Large quantities of the desired antibodies can also be obtained by multiplying mammalian cells *in vivo.* For this purpose, hybridoma cells producing the desired antibodies are injected into histocompatible mammals to cause growth of antibody-producing tumours. Optionally, the animals are primed with a hydrocarbon, especially mineral oils such as pristane (tetramethyl-pentadecane), prior to the injection. After one to three weeks, the antibodies are isolated from the body fluids of those mammals. For example, hybridoma cells obtained by fusion of suitable myeloma cells with antibody-producing spleen cells from Balb/c mice, or transfected cells derived from hybridoma cell line Sp2/0 that produce the desired antibodies are injected intraperitoneally into Balb/c mice optionally pre-treated with pristane, and, after one to two weeks, ascitic fluid is taken from the animals.

The foregoing, and other, techniques are discussed in, for example, Kohler and Milstein, (1975) Nature 256:495-497; US 4,376,110; Harlow and Lane, Antibodies: a Laboratory Manual, (1988) Cold Spring Harbor, incorporated herein by reference. Techniques for the preparation of recombinant antibody molecules are described in the above references and also in, for example, EP 0623679; EP 0368684 and EP 0436597, which are incorporated herein by reference.

The cell culture supernatants are screened for the desired antibodies, preferentially by immunofluorescent staining of cells expressing the desired target by immunoblotting, by an enzyme immunoassay, e.g. a sandwich assay or a dot-assay, or a radioimmunoassay.

For isolation of the antibodies, the immunoglobulins in the culture supernatants or in the ascitic fluid may be concentrated, e.g. by precipitation with ammonium sulphate, dialysis against hygroscopic material such as polyethylene glycol, filtration through selective membranes, or the like. If necessary and/or desired, the antibodies are purified by the customary chromatography methods, for example gel filtration, ionexchange chromatography, chromatography over DEAE-cellulose and/or (immuno) affinity chromatography, e.g. affinity chromatography with the target molecule or with Protein-A.

Antibodies generated according to the foregoing procedures may be cloned by isolation of nucleic acid from cells, according to standard procedures. Usefully, nucleic acids variable domains of the antibodies may be isolated and used to construct antibody fragments, such as scFv.

The invention therefore preferably employs recombinant nucleic acids comprising an insert coding for a heavy chain variable domain and/or for a light chain variable domain of antibodies. By definition such nucleic acids comprise coding single stranded nucleic acids, double stranded nucleic acids consisting of said coding nucleic acids and of complementary nucleic acids thereto, or these complementary (single stranded) nucleic acids themselves.

Chimeric antibodies may be prepared by replacing the constant region of a mouse antibody with human protein, while allowing the antibody's variable region to remain mouse protein. Several methods for the production of chimeric antibodies are known. By way of example, chimeric antibodies may be prepared in which constant regions of the heavy chain and the light chain of mouse monoclonal antibody are converted into human constant regions, are produced in animal cells making use of recombinant DNA techniques. Examples of such processes include a process in which humanised chimera antibody is produced using chromosomal DNA as a gene which encodes mouse H chain variable region (to be referred to as "V.sub.H " hereinafter) and L chain variable region (to be referred to as "V.sub.L " hereinafter) (Morrison et al., Proc. Natl. Acad. Sci. U.S.A., 81, 6851 (1984); Neuberger et al., Nature, 314, 268 (1985); Nishimura et al., Cancer Res., 47, 999 (1987); Dorai et al., J. Immunol., 139, 4232 (1987); Kameyama et al., FEBS letter, 244, 301 (1989)) and another process in which humanised chimera antibody is produced using cDNA (Gillies et al., J. Immunol. Methods, 125, 191 (1989); Liu et al., published International Application in Japan No. 2-501886).

Various methods may be used to prepare monoclonal antibodies with human character, which bypasses the need for an antibody-producing human cell line. For example, useful mouse monoclonal antibodies may be "humanised" by linking rodent variable regions and human constant regions (Winter, G. and Milstein, C. (1991) Nature 349, 293-299). This may reduce the human anti-mouse immunogenicity of the antibody but residual immunogenicity is retained by virtue of the foreign V-region framework. Moreover, the antigen-binding specificity is essentially that of the murine donor. CDR-grafting and framework manipulation (see, for example, EP 0239400) has improved and refined antibody manipulation to the point where it is possible to produce humanised murine antibodies which are acceptable for therapeutic use in humans. Humanised antibodies may be obtained using other methods well known in the art (for example as described in US-A-239400).

Furthermore, nucleic acids encoding a heavy chain variable domain and/or for a light chain variable domain of antibodies may be enzymatically or chemically synthesised nucleic acids having the authentic sequence coding for a naturally-occurring heavy chain variable domain and/or for the light chain variable domain, or a mutant thereof. A mutant of the authentic sequence is a nucleic acid encoding a heavy chain variable domain and/or a light chain variable domain of the above-mentioned antibodies in which one or more amino acids are deleted or exchanged with one or more other amino acids. Preferably said modification(s) are outside the CDRs of the heavy chain variable domain and/or of the light chain variable domain of the antibody. Such a mutant nucleic acid is also intended to be a silent mutant wherein one or more nucleotides are replaced by other nucleotides with the new codons coding for the same amino acid(s). Such a mutant sequence is also a degenerated sequence. Degenerated sequences are degenerated within the meaning of the genetic code in that an unlimited number of nucleotides are replaced by other nucleotides without resulting in a change of the amino acid sequence originally encoded. Such degenerated sequences may be useful due to their different restriction sites and/or frequency of particular codons which are preferred by the specific host, particularly yeast, bacterial or mammalian cells, to obtain an optimal expression of the heavy chain variable domain and/or a light chain variable domain.

Alternatively antibodies may be selected and generated using phage display technology. Methods for the construction of bacteriophage antibody display libraries and lambda phage expression libraries are well known in the art (McCafferty *et al.* (1990) supra; Kang et al. (1991) Proc. Natl. Acad. Sci. U.S.A., 88: 4363; Clackson et al. (1991) Nature, 352: 624; Lowman et al. (1991) Biochemistry, 30: 10832; Burton et al. (1991) Proc. Natl. Acad. Sci U.S.A., 88: 10134; Hoogenboom et al. (1991) Nucleic Acids Res., 19: 4133; Chang et al. (1991) J. Immunol., 147: 3610; Breitling et al. (1991) Gene, 104: 147; Marks *et al.* (1991) supra; Barbas *et al.* (1992) supra; Hawkins and Winter (1992) J. Immunol., 22: 867; Marks et al., 1992, J Biol. Chem., 267: 16007; Lemer et al. (1992) Science, 258: 1313, incorporated herein by reference).

Techniques for the preparation of bispecific antibodies, are for example described in the following reviews and the references cited therein: Winter & Milstein, (1991) Nature 349:293-299; Plueckthun (1992) Immunological Reviews 130:151-188; Wright et al., (1992) Crti. Rev. Immunol.12:125-168; Holliger, P. & Winter, G. (1993) Curr. Op. Biotechn. 4, 446-449; Carter, et al. (1995) J. Hematother. 4, 463-470; Chester, K.A. & Hawkins, R.E. (1995) Trends Biotechn. 13, 294-300; Hoogenboom, H.R. (1997) Nature Biotechnol. 15, 125-126; Fearon, D. (1997) Nature Biotechnol. 15, 618-619; Plückthun, A. & Pack, P. (1997) Immunotechnology 3, 83-105; Carter, P. & Merchant, A.M. (1997) Curr. Opin. Biotechnol. 8, 449-454; Holliger, P. & Winter, G. (1997) Cancer Immunol. Immunother. 45,128-130.

### Method for the specific delivery of agents to the interior of tumour cells

Many cytotoxins and other agents or components of agents are activated by cytoplasmic components for example cellular enzymes, and therefore internalisation of the agent is essential for their activity.

Thus in a further aspect, the present invention provides a 5T4 antibody linked to an agent for use in a method for the specific delivery of one or more agents to the interior of tumour cells. The method may comprise the step of treating the one or more cells with a 5T4 antibody linked to those one or more agents.

In the context of the present invention, the term 'treating' refers to the process of bringing one or more cells into contact with a specific 5T4 antibody linked to an agent. Suitable methods for 'treating' cells will depend upon whether the procedure is carried out *in vivo* or *in vitro.*

### (1) Agents

The agents include molecules, which affect the cell structure by interacting with one or more cytoskeletal elements.

In addition, agents may comprise nucleic acid capable of being expressed to form a gene product. Suitable nucleic acids may be incorporated within a vector. Suitable vectors will be familiar to those skilled in the art. The vector may be a phage based, plasmid based, and/or viral including retro-viral based.

If the vector is a viral vector then it may include, but is not limited to, adenovirus vector, an adeno-associated viral vector, a herpes viral vector, retroviral vector, lentiviral vector, baculoviral vector, pox viral vectors or parvovirus vectors (see Kestler et al 1999 Human Gene Ther 10(10):1619-32). In the case of viral vectors, delivery of the nucleotide sequence encoding the agent of the present invention may be mediated by viral infection. One skilled in the art will appreciate that this list is not intended to be exhaustive.

Preferably, the viral vector is a lentiviral vector. Lentiviruses are a subtype of retroviruses, that may represent an alternative to retroviruses. Lentiviruses, such as HIV, simian and feline immunodeficiency viruses, can infect non-dividing cells and integrate in the same way as other retroviruses.

Advantageously, the vector will be an expression vector and will include those additional elements necessary for expression of nucleic acid (for example promoter and enhancer sequences).

The nucleic acid for delivery into the interior of tumour cells may encode one or more molecules, which affect the structure and/or function of the tumour cell. Such molecules include enzymes, enzymes inhibitors, co-factors for enzymes or other proteins, protein sequestering agents, suicide genes or interfering RNAs. One skilled in the art will appreciate that this list is not intended to be exhaustive.

The nucleic acid for delivery into the interior of tumour cells may therefore encode one or more molecules which affect the structure and/or function of the tumour cell by inhibiting expression of a cellular component, for example at the level of transcription, transcript stability, translation or post-translational stability. For example, the molecule may be an antisense sequence or an siRNA. The inhibition of gene expression using antisense technology is well known.

Post-transcriptional gene silencing (PTGS) mediated by double-stranded RNA (dsRNA) is a conserved cellular defence mechanism for controlling the expression of foreign genes. It is thought that the random integration of elements such as transposons or viruses causes the expression of dsRNA, which activates sequence-specific degradation of homologous single-stranded mRNA or viral genomic RNA. The silencing effect is known as RNA interference (RNAi). The mechanism of RNAi involves the processing of long dsRNAs into duplexes of 21-25 nucleotide (nt) RNAs. These products are called small interfering or silencing RNAs (siRNAs) which are the sequence-specific mediators of mRNA degradation. In differentiated mammalian cells dsRNA >30bp has been found to activate the interferon response leading to shut-down of protein synthesis and non-specific mRNA degradation (Stark et al 1998). However this response can be bypassed by using 21nt siRNA duplexes (Elbashir et al 2001, Hutvagner et al 2001) allowing gene function to be analysed in cultured mammalian cells.

The nucleic acid for delivery into the interior of tumour cells may also encode one or more suicide genes which affect the structure and/or function of the tumour cell. A number of suicide gene systems have been identified, including, but not limited to, the herpes simplex virus thymidine kinase gene, the cytosine deaminase gene, the varicella-zoster virus thymidine kinase gene, the nitroreductase gene, the *Escherichia coli gpt* gene, and the *E*. *coli Deo* gene. Suicide genes in cancer therapy have been reviewed in, for example, World J Surg 2002 Jul;26(7):783-9, Adv Exp Med Biol 2000;465:411-22 and Semin Oncol. 1996 Feb;23(1):31-45.

The nucleic acid for delivery into the interior of tumour cells may also encode one or more nucleotide sequences that are capable of "knocking out" the expression of a particular gene which affects the structure and/or function of the tumour cell. There are several "knock out" strategies known in the art. For example, the nucleotide sequence may be capable of integrating in the genome of a cell so as to disrupt expression of a particular gene. Expression may be disrupted by, for example, introducing a premature stop codon, by rendering the downstream coding sequence out of frame, or by affecting the capacity of the encoded protein to fold (thereby affecting its function).

### 2) In vivo methods for treating cells with Agents

Generally agents as herein defined and the 5T4 antibody they are linked to are combined with one or more pharmaceutical carriers, diluents and/or excipients to form a composition for treating cells.

The agent or composition may be administered in a convenient manner such as by the oral, intravenous (where water soluble), intramuscular, subcutaneous, intranasal, intradermal or suppository routes or implanting (e.g. using slow release molecules). Depending on the route of administration, the agent may be required to be coated in a material to protect said ingredients from the action of enzymes, acids and other natural conditions which may inactivate said ingredient.

In order to administer the agent by other than parenteral administration, it will be coated by, or administered with, a material to prevent its inactivation. For example, the agent may be administered in an adjuvant, co-administered with enzyme inhibitors or in liposomes. Adjuvant is used in its broadest sense and includes any immune stimulating compound such as interferon. Adjuvants contemplated herein include resorcinols, non-ionic surfactants such as polyoxyethylene oleyl ether and n-hexadecyl polyethylene ether. Enzyme inhibitors include pancreatic trypsin.

Liposomes include water-in-oil-in-water CGF emulsions as well as conventional liposomes.

The agent-5T4 antibody complex may also be administered parenterally or intraperitoneally. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersion. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene gloycol, and the like), suitable mixtures thereof, and vegetable oils. The proper fluidity can be maintained, for example, by the use of a coating such as lecithin, by the maintenance of the required particle size in the case of dispersion and by the use of superfactants.

The prevention of the action of microorganisms can be brought about by various antibacterial and antifungal agents, for example, parabens, chlorobutanol, phenol, sorbic acid, thiomersal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compound in the required amount in the appropriate solvent with various amounts of the other ingredients enumerated above, as required, followed by filtered sterilisation. Generally, dispersions are prepared by incorporating the sterilised active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the agent-5T4 antibody is suitably protected as described above, it may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. The amount of active compound in such therapeutically useful compositions in such that a suitable dosage will be obtained.

The tablets, troches, pills, capsules and the like may also contain the following: a binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavouring agent such as peppermint, oil of wintergreen, or cherry flavouring. When the dosage unit form is a capsule, it may contain, in addition to materials of the above type, a liquid carrier.

Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound may be incorporated into sustained-release preparations and formulations.

As used herein "pharmaceutically acceptable carrier and/or diluent and/or exipient" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the agent-5T4 ligand of the present invention, use thereof in the agent compositions of the present invention is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such as active material for the treatment of disease in living subjects having a diseased condition in which bodily health is impaired.

The principal active ingredients are compounded for convenient and effective administration in effective amounts with a suitable pharmaceutically acceptable carrier in dosage unit form. In the case of compositions containing supplementary active ingredients, the dosages are determined by reference to the usual dose and manner of administration of the said ingredients.

The invention is further described, for the purposes of illustration only, in the following examples, which are in no way limiting of the invention.

### Examples

### Example 1-General Method

The aim of this study was to establish that a monoclonal antibody specific to hu5T4 (H8) or a single chain antibody 5T4scFvmycHis (scFv) is able to be internalised with 5T4 after they have bound 5T4 at the cell surface.

### Experimental Design

The basis of internalisation experiments is to observe the decrease of Ab bound 5T4 at the cell surface of the tumour cell line over time. In this study we detect internalisation of 5T4 by extracellular labelling of 5T4 using H8 or the scFv, 5T4scFvmycHis (this is recombinant scFv with a myc his tag). For internalisation by monoclonal anti-body (H8) we used anti-mouse IgG-FITC to label remaining 5T4:H8 conjugates that have not been internalised. For internalisation by scFv use mouse anti-HIS followed by anti-mouse IgG-FITC to label remaining 5T4:scFv conjugates at the cell surface. Detection of 5T4:Ab conjugates is performed by flow cytometry

A reduction in the level of labelled conjugates at the cell surface over time is indicative of the rate of internalisation of 5T4:Ab conjugates. To ensure that live cells were observed the exclusion dye TOPRO-3 was used. Only TOPRO-3 negative cells were assessed for the level of 5T4:Ab conjugates internalised.

### Example 2-Detailed Method

- Incubate 2.5x10⁵ cells in 0.5ml with saturating amounts of H8 or scFv at 4°C for 60 minutes.
- Wash cells with cold FACs staining buffer by adding 3ml to each tube on ice and spinning at 1500rpm for 5 minutes at 4°C.
- Decant supernatant and add 1ml of 37°C RPMI+10%FBS to all tubes (except t=0 tube where 1ml of cold RPMI was added),
- Tightly close caps of t=0 tubes and place immediately on ice
- Place remaining tubes in 37°C 5% CO₂ incubator with loose caps
- Return appropriate tubes to ice at these time points (hr): 0, 1, 2, 4
- When last tube collected spin down cells decant supernatant and wash by resuspending in 3ml ice cold FACS staining buffer and spin for 5 mins at 500*g.
- Decant supernatant.
- Add 100microlitres of FACS staining buffer containing diluted mouse anti-HIS (1:1000) to cells labelled with LscFv
- Incubate 30 minutes at 4°C
- Wash twice with PBS Azide (3ml each wash)
- Add 100µl of FACS staining buffer with diluted goat anti-mouse FITC diluted 1:30 (final volume will be approx 300µl) to all tubes.
- Incubate 30 minutes at 4°C
- Wash twice with PBS Azide (3ml each wash).
- Resuspend in 0.5ml PBS Azide keep on ice and add Topro-3 (500x stock) just before acquisition on flow cytometer

### Example 3-Results

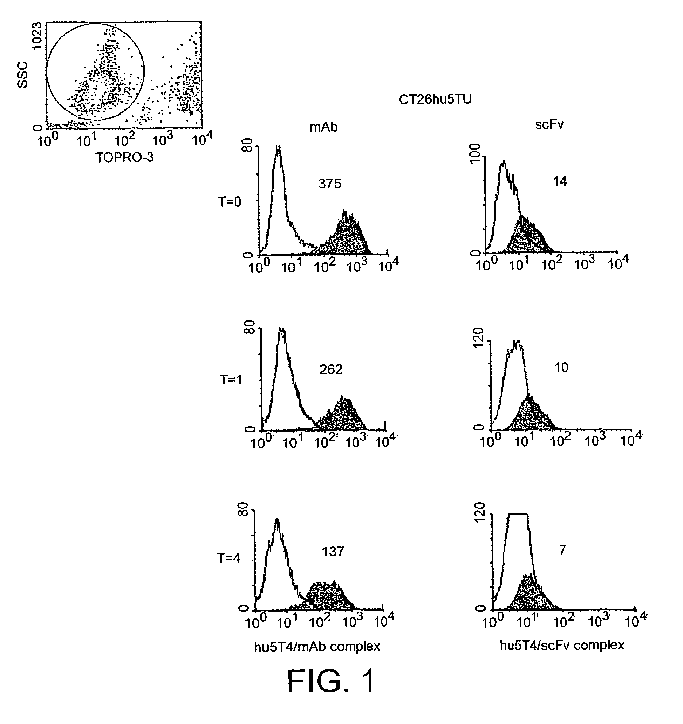

Figure 1: Internalisation of hu5T4 on the cell surface of the murine cell line CT26hu5T4.

Both the scFv and monoclonal antibody H8 are able to internalise with hu5T4 labelled at the cell surface of live cells. Within 4 hours at 37°C both the monoclonal anti-body and scFv are able to internalise with at least half of hu5T4 at the cell surface. H8 is more effective at intemalisation.

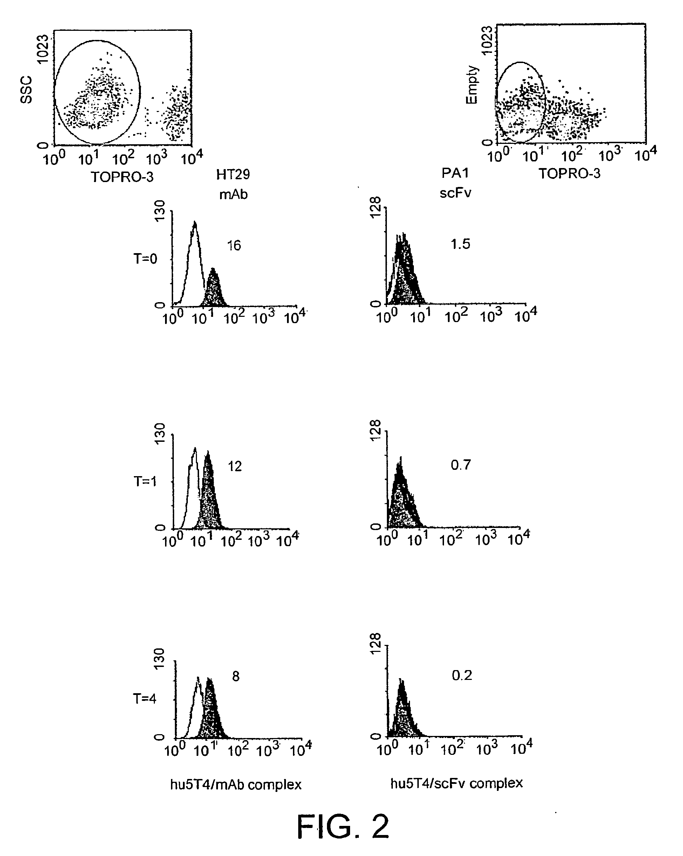

Figure 2: Internalisation of hu5T4 on the cell surface of the human cell line HT29 and PA1.

The monoclonal antibody, H8, is able to mediate internalisation of hu5T4 on the surface of the human cell line HT29. Again 50% of hu5T4 labelled by H8 was internalised within 4 hours of incubation at 37°C. The scFv is also able to internalise hu5T4 on the surface of another human cell line PA1. In this case half of hu5T4 at the cell surface was internalised within 1 hour of incubation at 37°C.

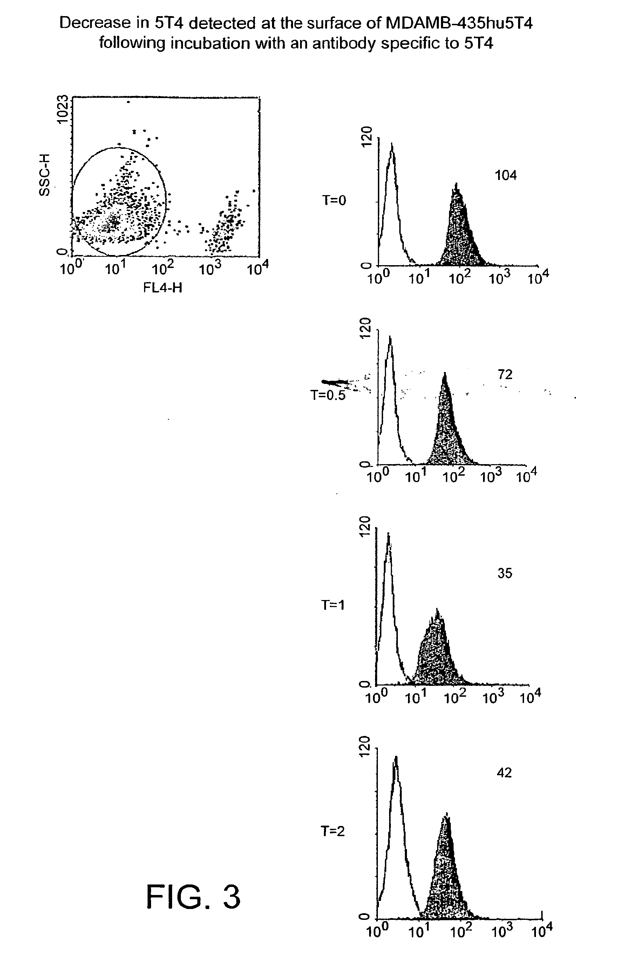

Figure 3: Internalisation of hu5T4 on the surface of MDAMB-4355T4

MDAMB-435 is a human breast tumour cell line that naturally expresses low levels of human 5T4. Stable transfectants of this cell line expressing high levels of 5T4 are prepared and are used to assess the internalisation of 5T4 on MDAMB-435 cells. Binding of H8 to 5T4 on this cell line results in a rapid reduction of 5T4 at the cell surface (almost 30% in 30 minutes). After 1-2 hours at 37 °C, a further 20-30% of labelled 5T4 is lost from the cell surface. This provides further evidence that human 5T4 is a suitable target for antibody mediated transport of molecules into human tumour cells.

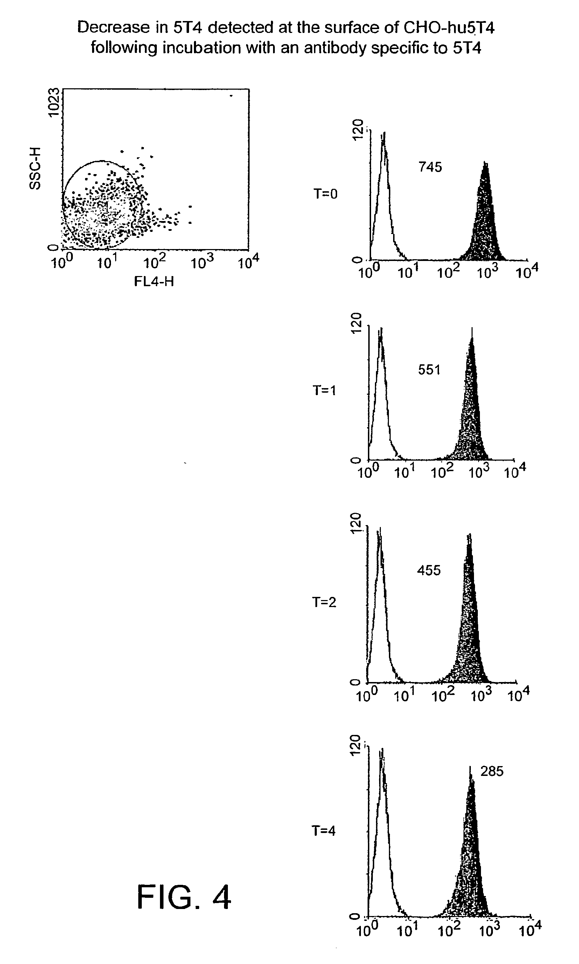

Figure 4: Internalisation of hu5T4 on the surface of CHO-5T4EKmycHIS

The Chinese hamster ovary cell line expressing human 5T4 is used as described previously. 5T4 protein expressed in this cell line contains a cleavage site for enterokinase followed by a myc-HIS tag at its C-terminus. It is determined whether this additional 26 amino acids forming the enterokinase recognition site and myc HIS tags prevent internalisation of 5T4 by H8. Figure 4 demonstrates that the internalisation properties of H8 and 5T4 are not lost when enterokinase and myc-HIS moieties are present at the C-terminus of 5T4 since more than 35% of labelled 5T4 is lost from the cell surface after 2 hours at 37 °C.

All publications mentioned in the above specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry, molecular biology and biotechnology or related fields are intended to be within the scope of the following claims.

## Claims

1. A 5T4 antibody linked to a cytotoxic agent, which, once internalised into the interior of a tumour cell, exerts one or more intracellular biological effects, wherein the cytotoxic agent comprises a molecule which affects the cell structure by interacting with one or more cytoskeletal elements.

2. A 5T4 antibody linked to an agent according to claim 1 wherein the 5T4Ab is a monoclonal antibody.

3. A 5T4 antibody linked to an agent according to claim 1 or 2 wherein the antibody is an scFv.

4. A 5T4 antibody linked to an agent according to claim 3 wherein the scFv is 5T4scFvmycHis.

5. A 5T4 antibody linked to an agent according to any one of the preceding claims wherein the 5T4 antibody is specific to human 5T4 antigen.

6. A 5T4 antibody linked to an agent according to claim 2 wherein the monoclonal antibody is H8.

7. A cell surface complex of a 5T4 antigen and a 5T4 antibody linked to an agent according to any preceding claim.

8. An internalised complex of a 5T4 antibody linked to an agent according to any of claims 1 to 6.

9. A 5T4 antibody linked to an agent according to any of claims 1 to 6 for use in a method for the internalisation of a 5T4 antibody linked to an agent.

10. A 5T4 antibody linked to an agent according to any of claims 1 to 6, for use in a method for the specific delivery of the agent to the interior of tumour cells.

11. A 5T4 antibody linked to an agent for use according to claim 10, wherein the delivery is into the cytoplasm.

12. A 5T4 antibody linked to an agent according to any of claims 1 to 6 for use in a method for decreasing the rate of proliferation of tumour cell/s.

13. A 5T4 antibody linked to an agent according to any of claims 1 to 6 for use in a method for the prophylaxis or treatment of cancer in a mammal.

## Patentansprüche

1. Mit einem zytotoxischen Mittel konjugierter 5T4-Antikörper, der, sobald er in das Innere einer Tumorzelle internalisiert ist, eine oder mehrere intrazelluläre biologische Auswirkungen bewirkt, wobei das zytotoxische Mittel ein Molekül umfasst, das die Zellstruktur dadurch beeinflusst, dass es mit einem oder mehreren Elementen des Zytoskeletts interagiert.

2. Mit einem Agens konjugierter 5T4-Antiköprer nach Anspruch 1, wobei es sich bei dem 5T4Ab um einen monoklonalen Antikörper handelt.

3. Mit einem Agens konjugierter 5T4-Antikörper nach Anspruch 1 oder 2, wobei es sich bei dem Antikörper um ein scFv handelt.

4. Mit einem Agens konjugierter 5T4-Antikörper nach Anspruch 3, wobei es sich bei dem scFv um 5T4scFvmycHis handelt.

5. Mit einem Agens konjugierter 5T4-Antikörper nach einem der vorhergehenden Ansprüche, wobei der 5T4-Antikörper für Human-5T4-Antigen spezifisch ist.

6. Mit einem Agens konjugierter 5T4-Antikörper nach Anspruch 2, wobei es sich bei dem monoklonalen Antikörper um H8 handelt.

7. Zelloberflächenkomplex aus einem 5T4-Antigen und einem mit einem Agens konjugierten 5T4-Antikörper nach einem vorhergehenden Anspruch.

8. Internalisierter Komplex eines mit einem Agens konjugierten 5T4-Antikörpers nach einem der Ansprüche 1 bis 6.

9. Mit einem Agens konjugierter 5T4-Antikörper nach einem der Ansprüche 1 bis 6 für die Verwendung in einem Verfahren zur Internalisierung eines mit einem Agens konjugierten 5T4-Antikörpers.

10. Mit einem Agens konjugierter 5T4-Antikörper nach einem der Ansprüche 1 bis 6 für die Verwendung in einem Verfahren für die spezifische Abgabe des Agens an das Innere von Tumorzellen.

11. Mit einem Agens konjugierter 5T4-Antikörper für die Verwendung nach Anspruch 10, wobei die Abgabe in das Zytoplasma erfolgt.

12. Mit einem Agens konjugierter 5T4-Antikörper nach einem der Ansprüche 1 bis 6 für die Verwendung in einem Verfahren zur Verringerung der Proliferenzrate einer Tumorzelle bzw. von Tumorzellen.

13. Mit einem Agens konjugierter 5T4-Antikörper nach einem der Ansprüche 1 bis 6 für die Verwendung in einem Verfahren für die Prophylaxe oder Behandlung von Krebs bei einem Säugetier.

## Revendications

1. Anticorps 5T4 lié à un agent cytotoxique, qui, une fois internalisé à l'intérieur d'une cellule tumorale, exerce un ou plusieurs effets biologiques intracellulaires, l'agent cytotoxique comprenant une molécule qui affecte la structure cellulaire en interagissant avec un ou plusieurs éléments cytosquelettiques.

2. Anticorps 5T4 lié à un agent selon la revendication 1, l'anticorps 5TA étant un anticorps monoclonal.

3. Anticorps 5T4 lié à un agent selon la revendication 1 ou 2, l'anticorps étant un scFv.

4. Anticorps 5T4 lié à un agent selon la revendication 3, le scFv étant 5T4scFvmycHis.

5. Anticorps 5T4 lié à un agent selon l'une quelconque des revendications précédentes, l'anticorps 5T4 étant spécifique à un antigène 5T4 humain.

6. Anticorps 5T4 lié à un agent selon la revendication 2, l'anticorps monoclonal étant H8.

7. Complexe de surface cellulaire d'un antigène 5T4 et d'un anticorps 5T4 lié à un agent selon l'une quelconque des revendications précédentes.

8. Complexe internalisé d'un anticorps 5T4 lié à un agent selon l'une quelconque des revendications 1 à 6.

9. Anticorps 5T4 lié à un agent selon l'une quelconque des revendications 1 à 6 pour utilisation dans un procédé pour l'internalisation d'un anticorps 5T4 lié à un agent.

10. Anticorps 5T4 lié à un agent selon l'une quelconque des revendications 1 à 6 pour utilisation dans un procédé pour l'administration spécifique de l'agent à l'intérieur de cellules tumorales.

11. Anticorps 5T4 lié à un agent pour utilisation selon la revendication 10, l'administration étant dans le cytoplasme.

12. Anticorps 5T4 lié à un agent selon l'une quelconque des revendications 1 à 6 pour utilisation dans un procédé pour diminuer le taux de prolifération de cellules tumorales.

13. Anticorps 5T4 lié à un agent selon l'une quelconque des revendications 1 à 6 pour utilisation dans un procédé pour la prophylaxie ou le traitement du cancer chez un mammifère.
